# EUROPÄISCHE PATENTSCHRIFT

(11) **EP 1 869 448 B1**
(45) Veröffentlichungstag und Bekanntmachung des Hinweises auf die Patenterteilung: **05.11.2008**
(21) Anmeldenummer: 06707841.0
(22) Anmeldetag: 25.01.2006
(51) Int. Cl.: G01N 33/28

(54) **VERFAHREN ZUR QUANTITATIVEN BESTIMMUNG EINES ALTERUNGSEINFLUSSES AUF EIN MOTORÖL**
METHOD FOR THE QUANTITATIVE DETERMINATION OF THE EFFECTS OF AGEING ON MOTOR OIL
PROCEDE DE DETERMINATION QUANTITATIVE D'UN EFFET DU VIEILLISSEMENT SUR UNE HUILE MOTEUR

(30) Priorität: 18.03.2005 DE 102005012454
(43) Veröffentlichungstag der Anmeldung: 26.12.2007
(73) Patentinhaber: ROBERT BOSCH GMBH, 70442 Stuttgart (DE)
(72) Erfinder: HALLER, Volker, 71088 Holzgerlingen (DE); NIEMANN, Markus, 66701 Beckingen (DE); HILBERATH, Thomas, 72800 Eningen U.A. (DE); HAMM, Gerald, 71083 Herrenberg (DE); SPEICHER, Rolf, 72072 Tuebingen (DE); SCHERER, Monika, 53225 Bonn (DE); KAESS, Udo, 70599 Stuttgart (DE)
(86) Internationale Anmeldenummer: PCT/EP2006/050446
(87) Internationale Veröffentlichungsnummer: WO 2006/097381

(56) Entgegenhaltungen:
- EP-A- 0 866 428
- WANG S S: "Road tests of oil condition sensor and sensing technique" SENSORS AND ACTUATORS B, Bd. 73, Nr. 2-3, 10. März 2001 (2001-03-10), Seiten 106-111, XP004317265 ISSN: 0925-4005
- WANG S S: "Engine oil condition sensor: method for establishing correlation with total acid number" SENSORS AND ACTUATORS B, Bd. 86, Nr. 2-3, 20. September 2002 (2002-09-20), Seiten 122-126, XP004380179 ISSN: 0925-4005

## Beschreibung

### STAND DER TECHNIK

Die vorliegende Erfindung betrifft ein Verfahren zur quantitativen Bestimmung mindestens eines Alterungseinflusses auf ein Motoröl.

Ein Verfahren gemäß dem Oberbegriff des Anspruchs 1 ist aus der Druckshrift Sensors & Actuators B, Bd. 73, Nr. 2-3, Seiten 106-111 bekannt.

Obwohl nachfolgend die Erfindung mit Bezug auf die quantitative Bestimmung von Einflüssen auf eine Oxidation oder eine Versauerung eines Motoröl beschrieben wird, ist die Erfindung nicht darauf beschränkt, sondern betrifft allgemein Verfahren, welche Alterungseinflüsse auf ein Motoröl bestimmen.

In Verbrennungsmotoren werden zur Schmierung der beweglichen Teile Motoröle eingesetzt, um die Reibung und den Verschleiß von zueinander bewegten Metalloberflächen zu verringern. Das Motoröl unterliegt mehreren Alterungsprozessen, welche dazu führen, dass das Öl nach einer gewissen Zeit gewechselt werden muss. Hierbei ist es von großem Interesse, eine Sensoreinrichtung und ein Verfahren bereitzustellen, welches den Zustand des Öls in einem Verbrennungsmotor im laufenden Betrieb erfassen kann.

Der Zustand des Motoröls wird in verschiedener Weise durch vielfältigen Alterungsprozesse beeinflusst. Die Wirkung einiger Alterungsprozesse auf die Eigenschaften und Zusammensetzung des Motoröls ist bekannt. Zudem lassen sich diese Alterungsprozesse mit bestimmten Vorgängen in einem Motor bzw. an den Motor angeschlossener Einrichtungen identifizieren. Daher können im Umkehrschluss aus dem Zustand des Öl mögliche Fehleinstellungen oder Defekte des Motor bzw. der angeschlossenen Einrichtungen erfasst werden.

Zwei Alterungseinflüsse sind die Abwärme des Motors und der Zustrom von Luftsauerstoff, welche zu einer Oxidation und einer Versauerung des Öl führen. Die Oxidation führt zu einer erhöhten Viskosität des Öls. Die Bestimmung der Viskosität ermöglicht somit eine Bestimmung des Grades der Oxidation. Da Carbonsäuren durch Oxidation des Motoröls entstehen und damit die Menge der Carbonsäuren abhängig von dem Oxidationsgrad sind, kann über die Viskosität auch der Anteil dieser Säuren in dem Öl erfasst werden. Dieses sehr einfache Untersuchungsverfahren berücksichtigt jedoch nicht weitere Einflüsse, welche zu einer Erhöhung der Viskosität des Öls beitragen, ohne den Säuregehalt zu verändem, wie zum Beispiel eine verstopfte Abgasnachbehandlungseinrichtung, welche den Russgehalt in dem Motor erhöht.

Zudem wird die Menge von Durchblasegasen nicht erfasst, da diese nur zu einer Erhöhung des Säureanteils führen ohne dass dies unmittelbar durch die Viskosität erfassbar ist.

### VORTEILE DER ERFINDUNG

Das erfindungsgemäße Verfahren zur quantitativen Bestimmung eines Alterungseinflusses auf ein Motoröl sieht folgende Schritte vor: Erfassen zweier Größen des Motoröls, wobei die eine der beiden physikalischen Größen die Viskosität und die andere Größe der Säuregehalt des Motoröls ist; Bestimmen einer ersten Abweichung der erfassten ersten Größe zu einem ersten Vorgabewert; Schätzen der zweiten Größe basierend auf dem Unterschied; Bestimmen einer zweiten Abweichung der geschätzten zweiten Größe von der erfassten zweiten Größe als Maß für den Alterungseinfluss auf das Motoröl.

Ein Vorteil des vorliegenden Verfahrens ist, dass die Einflüsse gezielt erfasst werden können, welche nur auf den Säuregehalt einwirken oder die Viskostät des Öls verändern.

In den Unteransprüchen finden sich vorteilhafte Weiterbildungen und Verbesserungen des in Patentanspruch 1 angegebenen Verfahrens.

Gemäß einer Weiterbildung wird in einem ersten Fall, wenn die geschätzte zweite Größe größer als die erfasste zweite Größe ist, der Alterungseinfluss als ein erster Alterungseinfluss identifiziert, welcher die erste Größe beeinflusst und im gegenteiligen zweiten Fall, der Alterungseinfluss als ein zweiter Alterungseinfluss identifiziert, welcher die zweite Größe beeinflusst.

Gemäß einer Weiterbildung werden als einer der Alterungseinflüsse eine Zustrommenge von Durchblasegasen und/oder ein Schwefelanteil in dem Motoröl bestimmt, welche den Säuregehalt beeinflussen oder als der andere Alterungseinfluss eine Zustrommenge von Rußpartikeln und/oder ein Nitrationsvorgang des Öls bestimmt, welche die Viskosität beeinflussen.

Gemäß einer Weiterbildung wird als eine dritte Größe die Permittivität oder der spezifische Widerstand des Motoröls erfasst und basierend auf dieser Größe der Russgehalt des Motoröls bestimmt.

Gemäß einer Weiterbildung wird eine Temperatur der Motoröls bestimmt, der Vorgabewert von der Temperatur abhängig gewählt und die Schätzung der zweiten Größe erfolgt basierend auf der Temperatur der Motoröls.

Gemäß einer Weiterbildung wird eine Verwendungsdauer des Motoröls aufgezeichnet und der Vorgabewert abhängig von der Verwendungsdauer gewählt.

Ausführungsbeispiele der Erfindung sowie vorteilhafte Weiterbildungen werden mit Bezug auf die Figuren der schematischen Zeichnungen dargestellt und in der nachfolgenden Beschreibung näher erläutert.

### ZEICHNUNGEN

In den Figuren zeigen:
- Fig. 1: ein Blockdiagramm einer Ausführungsform der vorliegenden Erfindung; und
- Fig. 2: eine graphische Darstellung zur Erläuterung einer ersten Ausführungsform der vorliegen- den Erfindung.

### BESCHREIBUNG DER AUSFÜHRUNGSBEISPIELE

In den Figuren bezeichnen gleiche Bezugszeichen gleiche oder funktionsgleiche Merkmale, soweit nichts Gegenteiliges angegeben ist.

Untersuchungen von Motorölen werden heutzutage verwendet, um den Zustand eines Motors zu bestimmen. Vielfältige Charakteristika eines Motors nehmen in unterschiedlicher Weise Einfluss auf den Zustand eines Öls. Standardmäßig wird durch Zufuhr von Luftsauerstoff und der Abwärme des Motors das Motoröl oxidiert. Hierbei entstehen durch die Oxidation u.a. Aldehyde, Ketone und Carbonsäuren. Diese können in weiteren chemischen Reaktionen zu schlammartigen, größtenteils ölunlöslichen Ablagerungen weiterreagieren und sich an Metalloberflächen festsetzen. Andere Alterungsprodukte des Öls sind flüssig und bewirken einen Viskositätsanstieg. Auf Basis von Laboruntersuchungen ist der Grad der Oxidation des Motoröls und damit dessen Viskosität bei typischen Belastungen des Motors in Abhängigkeit der Verwendungsdauer des Motoröls bekannt. Ergeben sich hierbei erhebliche Unterschiede zu den erwarteten Werten, insbesondere eine deutlich erhöhte Viskosität, so lässt dies auf eine Fehlfunktion des Motors, der Ölfilter, von Abgasnachbehandlungseinrichtungen oder Ähnlichem schließen.

Ein weiterer Grund die Viskosität des Öls zu überwachen ist, dass ab einem kritischen Wert der Viskosität ein erhöhter Abrieb und Verschleiß der Elemente auftritt. Unterschreitet die Viskosität des Öls auf der anderen Seite einen kritischen Wert, kann es zum Abreißen des schützenden Schmierfilms zwischen den Teilen führen, was im schlimmsten Fall ein "Festfressen" des Motors verursacht. Daher muss das Motoröl vor Erreichen dieser Viskositätswerte ausgetauscht werden. Durch Vorgabe einer maximalen Kilometerzahl wird mit einer sehr großen Sicherheitsmarge erreicht, dass die Viskosität unterhalb des kritischen Werts bleibt. Jedoch wird hierbei nachteiligerweise in der Regel das Öl sehr viel früher gewechselt, als es notwendig wäre. Durch permanente Überwachung der Viskosität kann die Sicherheitsmarge reduziert werden und das Öl somit im Mittel länger verwendet werden.

Ein weiteres Kriterium, welches einen Ölwechsel zwingend notwendig macht, ist ein zu hoher Gehalt an Säuren im Öl. Durch Zugabe von basischen Additiven werden die Säuren, z.B. die Carbonsäuren, in dem Motoröl gepuffert. Durch die Säuren verstärkt sich die Korrosion, insbesondere von Buntmetallen. Außerdem wirken die Säuren in dem Öl katalytisch auf die Oxidation des Öls. Daher ist in einer ersten Näherung der Säuregehalt des Motoröls proportional zu der Oxidation und damit linear von der Viskosität des Motoröls abhängig.

Daher wird als Hilfsgröße für die Bewertung des Säuregehalts die Viskosität herangezogen. Allerdings führen auch Durchblasegase (Blow-by-Gase) und Schwefel aus dem Kraftstoff zur zusätzlichen Bildung von Säuren unabhängig von der Oxidation. Durch eine Analyse der Viskosität des Motoröls kann somit kein Rückschluss auf den Beitrag dieser Durchblasegase bzw. des zugeführten Schwefels gezogen werden. Jedoch ist eine Erfassung von untypisch hohen Durchblasgasen interessant, da diese z.B. auf Ablagerungen an Kolbenringen und einer suboptimalen Abdichtung des Kurbelgehäuses hinweisen. Zudem zeigt ein Anstieg des Säuregehalts an, dass die basischen Additive in dem Motoröl aufgebraucht sind. Ab dem Zeitpunkt, an dem die basischen Additive aufgebraucht sind, erhöht sich der Oxidationsgrad des Motoröls und damit auch die Viskosität des Motoröls exponentiell. Daher ist ab diesem Zeitpunkt ein zeitnaher Ölwechsel dringend anzuraten.

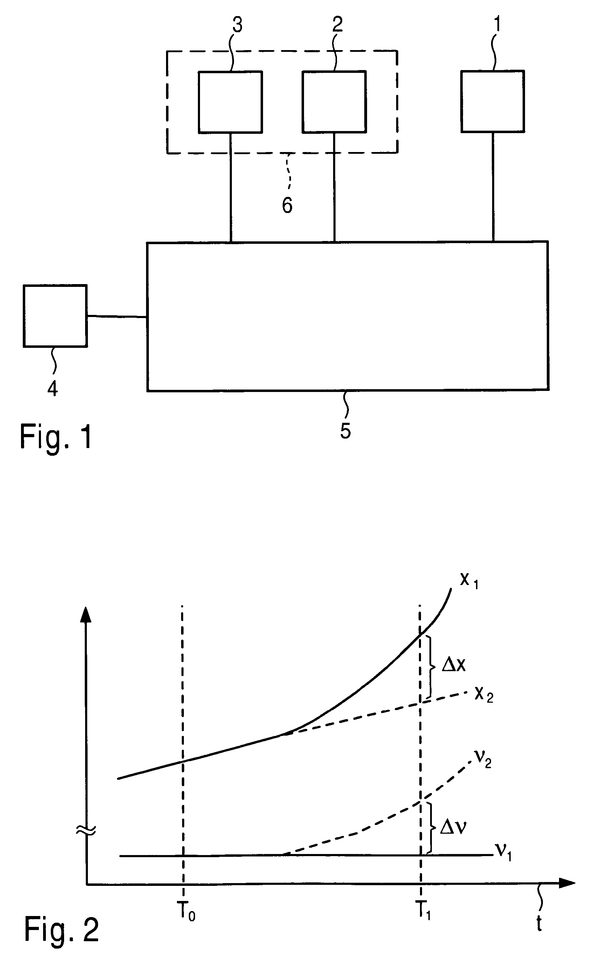

In Fig. 1 ist eine Ausführungsform der vorliegenden Erfindung dargestellt. In dem zu untersuchenden Motoröl sind ein Säuresensor 1 und ein Viskositätssensor 2 eingetaucht. Zusätzlich kann noch ein Permittivitätssensor 3, welcher vorzugsweise als kombinierter Viskositäts/Pemvttivitäts-Sensor ausgebildet ist, in das Motoröl getaucht werden. Die Signale von den Sensoren werden von einer Datenverarbeitungseinrichtung 5 bearbeitet. Zusätzlich ist es von Vorteil, eine Verwendungsdauer-Erfassungseinrichtung 4 bereitzustellen, welche bestimmt, wie lange das Motoröl bereits verwendet wird, welchen Maximaltemperaturen das Öl ausgesetzt war, welcher mittlerer Wärme das Motoröl ausgesetzt war und bei welchen Drehzahlen der Motor betrieben wurde oder weitere erfasste Motorparameter. Diese Daten können alle oder teilweise der Datenverarbeitungseinrichtung 5 zur Auswertung der Signale der Viskositäts-, Permittivitäts- und Säuresensoren zur Verfügung gestellt werden.

In Fig. 2 ist zur Erläuterung eines Auswerteverfahrens der Datenverarbeitungseinrichtung 5 dargestellt. Über die Zeit t sind die Viskosität ν und der Säuregehalt x eines Motoröls dargestellt. Die Größen können durch die entsprechenden Sensoreinrichtungen 1, 2, 3 zu einem Zeitpunkt T₀, T₁ erfasst werden. Zu einem ersten Zeitpunkt T₀ erfasst der Säuresensor 1 den Säuregehalt x₁ und der Viskositätssensor die Viskosität ν₁. In der Datenverarbeitungseinrichtung 5 ist ein Modell bereitgestellt, welches basierend auf dem Säuregehalt x₁ die Viskosität ν₁ schätzen kann, unter der Annahme, dass nur die Oxidation des Motoröls zur Bildung von Säuren, insbesondere der Carbonsäuren, führt. Durch ein Vergleichen der geschätzten Viskosität ν₂ mit der erfassten Viskosität ν₁ ergibt sich zum Zeitpunkt T₀ kein Unterschied. D.h., dass keine weiteren Einflüsse auf das Motoröl einwirken, welche zu einer Erhöhung der Viskosität oder des Säuregehalts in dem Motoröl führen.

Zu dem Zeitpunkt T₁ ist beispielhaft das Verhalten des Motoröls dargestellt, wenn sich der Säuregehalt z.B. aufgrund von Durchblasegasen stark erhöht. In einem ersten Schritt wird ein Unterschied Δx oder eine Abweichung des erfassten Säuregehalts x₁ zu einem Vorgabewert x₂ oder erwarteten Säuregehalt x₂ ermittelt. Der Vorgabewert kann einerseits der Säuregehalt eines neuen Motoröls sein, oder bereits einen Oxidationsgrad und damit verbundener Säureanteil eines Motoröls berücksichtigen, welcher basierend auf der Verwendungsdauer, der Maximaltemperaturen, der mittleren Temperatur und der Drehzahlen des Motors durch die Datenverarbeitungseinrichtung bestimmt wird. Basierend auf der Abweichung Δx wird eine Viskosität ν₂ bestimmt. Dies kann in einfacher Weise dadurch geschehen, dass eine proportionale Abhängigkeit der Viskosität von dem Säuregehalt angenommen wird und entsprechend dem Vorgabewert des Säuregehalts auch ein Vorgabewert für die Viskosität bereitgehalten wird. Danach wird die auf diese Weise geschätzte Viskosität ν₂ mit der erfassten Viskosität ν₁ verglichen. Ergibt sich hierbei eine Abweichung Δν, so ist diese auf zusätzliche Einflusse (keine reine Oxidation aufgrund von Wärme und Luftzufuhr und Bildung der Carbonsäuren) zurückzuführen. Ist die geschätzte Viskosität ν₂, wie in Fig. 2 dargestellt, größer als die erfasste Viskosität ν₁, so liegt die Ursache darin, dass ein Einfluss die Viskosität erhöht hat, ohne gleichzeitig den Säuregehalt zu erhöhen. Ein solcher Einfluss sind Rußpartikel, welche u.a. durch Abgasnachbehandlungseinrichtungen in diesen Motoren verstärkt in das Motoröl gelangen. Ein untypisch großer Unterschied Δν der erfassten Viskosität ν₁ zu der geschätzten Viskosität ν₂ würde hierbei zum Anlass genommen, die Abgasnachbehandlungseinrichtung zu reinigen. Der zweite gegenteilige Fall, also wenn die geschätzte Viskosität ν₂ geringer als die erfasste Viskosität ν₁ ist, ergibt sich bei Bildung von Säuren, , welche nicht durch Oxidation von Motoröl entstehen. Diese weiteren Säuren entstehen u.a. durch Schwefel, welche durch Kraftstoff in das Motoröl gelangt und auch durch Durchblasegase. Ein untypisch hoher Wert in der Abweichung Δν weist u.a. auf Ablagerungen an Kolbenringen hin.

Als zusätzliche Größe kann die Permittivität und/oder der elektrische spezifische Leitwert des Motoröls bestimmt werden. Durch diese Größe ist insbesondere ein Rückschluss auf den Rußanteil in dem Motoröl möglich.

### BEZUGSZEICHENLISTE

- 1: Säuresensor
- 2: Viskositätssensor
- 3: Permittivitätssensor
- 4: Erfassungseinrichtung
- 5: Datenverarbeitungseinrichtung
- t: Zeit
- ν: Viskosität
- x: Säuregehalt
- T₀, T₁: Zeitpunkte
- x₁: erfasster Säuregehalt
- ν₁: erfasste Viskosität
- ν₂: geschätzte Viskosität
- x₂: Vorgabewert
- Δx: Abweichung
- Δν: Abweichung

## Patentansprüche

1. Verfahren zur quantitativen Bestimmung mindestens eines Alterungseinflusses auf ein Motoröl mit den folgenden Schritten:
Erfassen zweier Größen des Motoröls, wobei die eine der beiden physikalischen Größen die Viskosität (ν1) und die andere Größe der Säuregehalt (x1) des Motoröls ist;
und **gekennzeichnet durch**
Bestimmen einer ersten Abweichung (Δx) der erfassten ersten Größe (x1) zu einem ersten Vorgabewert (x2);
Schätzen der zweiten Größe (ν2) basierend auf dem Unterschied (Δx);
Bestimmen einer zweiten Abweichung (Δν) der geschätzten zweiten Größe (ν2) von der erfassten zweiten Größe (ν1) als Maß für den Alterungseinfluss auf das Motoröl.

2. Verfahren nach Anspruch 1, **dadurch gekennzeichet, dass** im ersten Fall, wenn die geschätzte zweite Größe (ν2) größer als die erfasste zweite Größe (ν1) ist, der Alterungseinfluss als ein erster Alterungseinfluss identifiziert wird, welcher die erste Größe (x1) beeinflusst und im gegenteiligen zweiten Fall, der Alterungseinfluss als ein zweiter Alterungseinfluss identifiziert wird, welcher die zweite Größe (ν1) beeinflusst.

3. Verfahren nach Anspruch 1 oder 2, **dadurch gekennzeichnet, dass** als einer der Alterungseinflüsse eine Zustrommenge von Durchblasegasen und/oder ein Schwefelanteil in dem Motoröl bestimmt werden, welche den Säuregehalt (x1) beeinflussen oder als der andere Alterungseinfluss eine Zustrommenge von Rußpartikeln und/oder ein Nitrationsvorgang des Öls bestimmt werden, welche die Viskosität (ν1) beeinflussen.

4. Verfahren nach mindestens einem der vorhergehenden Ansprüche, **dadurch gekennzeichnet, dass** als eine dritte Größe die Permittivität oder der spezifische Widerstand des Motoröls erfasst wird und basierend auf dieser Größe der Russgehalt des Motoröls bestimmt wird.

5. Verfahren nach mindestens einem der vorhergehenden Ansprüche, **dadurch gekennzeichnet, dass** eine Temperatur der Motoröls bestimmt wird, der Vorgabewert (x2) von der Temperatur abhängig gewählt wird und die Schätzung der zweiten Größe (v2) basierend auf der Temperatur der Motoröls erfolgt.

6. Verfahren nach mindestens einem der vorhergehenden Ansprüche, **dadurch gekennzeichnet, dass** eine Verwendungsdauer (T1) des Motoröls aufgezeichnet wird und der Vorgabewert (x2) abhängig von der Verwendungsdauer (T1) gewählt wird.

## Claims

1. Method for the quantitative determination of at least one ageing influence on an engine oil, having the following steps:
Detection of two variables of the engine oil, one of the two physical variables being the viscosity (v1) and the other variable the acid content (x1) of the engine oil;
and **characterized by**
determination of a first deviation (Δx) of the detected first variable (x1) with respect to a first stipulated value (x2);
estimation of the second variable (ν2) based on the difference (Δx);
determination of a second deviation (Δν) of the estimated second variable (ν2) from the detected second variable (ν1) as a measure of the ageing influence on the engine oil.

2. Method according to Claim 1, **characterized in that,** in the first case, when the estimated second variable (ν2) is higher than the detected second variable (ν1), the ageing influence is identified as a first ageing influence which influences the first variable (x1), and, in the contrary second case, the ageing influence is identified as a second ageing influence which influences the second variable (ν1).

3. Method according to Claim 1 or 2, **characterized in that,** as one of the ageing influences, an inflow quantity of blow-through gases and/or a sulphur fraction in the engine oil are determined, which influence the acid content (x1), or, as the other ageing influence an inflow quantity of soot particles and/or a nitration process of the oil are determined, which influence the viscosity (ν1).

4. Method according to at least one of the preceding claims, **characterized in that,** as a third variable, the permittivity or the specific resistance of the engine oil is detected, and, based on this variable, the soot content of the engine oil is determined.

5. Method according to at least one of preceding claims, **characterized in that** a temperature of the engine oil is determined, the stipulated value (x2) is selected as a function of the temperature, and the estimation of the second variable (ν2) takes place, based on the temperature of the engine oil.

6. Method according to at least one of the preceding claims, **characterized in that** a period of use (T1) of the engine oil is recorded, and the stipulated value (x2) is selected as a function of the period of use (T1).

## Revendications

1. Procédé de détermination quantitative d'au moins une influence de vieillissement sur une huile moteur, comprenant les étapes suivantes :
- saisie de deux grandeurs de l'huile moteur, l'une des deux grandeurs physiques étant la viscosité (v1) et l'autre grandeur la teneur en acide (x1) de l'huile moteur,
**caractérisé en ce qu'**
- on détermine un premier écart (Δx) de la première grandeur (x1) saisie par rapport à une première valeur prédéfinie (x2);
- on évalue la seconde grandeur (v2) en fonction de l'écart (Δx),
- on détermine un second écart (Δv) de la seconde grandeur évaluée (v2) par rapport à la seconde grandeur saisie (v1) comme mesure de l'influence du vieillissement sur l'huile moteur.

2. Procédé selon la revendication 1,
**caractérisé en ce que**
dans le premier cas, si la seconde grandeur évaluée (v2) est supérieure à la seconde grandeur saisie (v1), l'influence du vieillissement est identifiée comme une première influence de vieillissement qui agit sur la première grandeur (x1) et dans le cas contraire, on identifie l'influence du vieillissement comme seconde influence du vieillissement qui agit sur la seconde grandeur (v2).

3. Procédé selon la revendication 1 ou 2,
**caractérisé en ce que**
comme l'une des influences du vieillissement, on définit le débit de passage de bulles de gaz qui et/ou la teneur en soufre de l'huile moteur, influençant la teneur en acidité (x1) ou comme autre influence du vieillissement, l'arrivée de particules de suie et/ou une phase de nitratation de l'huile qui influence la viscosité.

4. Procédé selon les revendications précédentes,
**caractérisé en ce que**
comme troisième grandeur, on saisit la permittivité ou la résistance spécifique de l'huile moteur et en fonction de cette grandeur, on définit la teneur en suie de l'huile moteur.

5. Procédé selon les revendications précédentes,
**caractérisé en ce qu'**
on détermine une température de l'huile moteur, on choisit la valeur prédéfinie (x2) de la température en fonction de la température de l'huile moteur et on évalue la seconde grandeur (v2) en fonction de la température de l'huile moteur.

6. Procédé selon les revendications précédentes,
**caractérisé en ce qu'**
on enregistre une durée d'utilisation (T1) de l'huile moteur et on choisit la valeur prédéfinie (x2) en fonction de la durée d'utilisation (T1).
